# EUROPEAN PATENT SPECIFICATION

(11) **EP 2 970 821 B1**
(45) Date of publication and mention of the grant of the patent: **13.02.2019**
(21) Application number: 14764531.1
(22) Date of filing: 17.03.2014
(51) Int. Cl.: C07C 219/12, C07D 219/08, C08K 5/17, C07C 219/08

(54) **BIO-BASED DISPERSANTS**
DISPERGIERMITTEL AUF BIOBASIS
DISPERSANTS À BASE BIOLOGIQUE

(30) Priority: 15.03.2013 US 201361786719 P
(43) Date of publication of application: 20.01.2016
(73) Proprietor: Ethox Chemicals, LLC, Greenville, SC 29605 (US)
(72) Inventor: TANNER, James, T., Greer, SC 29650 (US)
(74) Representative: Schüssler, Andrea
(86) International application number: PCT/US2014/030835
(87) International publication number: WO 2014/145972

(56) References cited:
- WO-A2-2013/126916
- CA-A1- 2 299 857
- US-A- 2 033 131
- US-A- 3 412 053
- US-A- 5 385 616
- US-A- 5 582 792
- US-A- 5 759 485
- US-A1- 2009 065 736
- US-A1- 2009 209 441
- US-A1- 2012 238 692
- US-B2- 6 723 785
- K CHOWDHURY ET AL: "Studies on the Fatty Acid Composition of Edible Oil", BANGLADESH JOURNAL OF SCIENTIFIC AND INDUSTRIAL RESEARCH., vol. 42, no. 3, 11 February 2008 (2008-02-11), XP055248819, BD ISSN: 0304-9809, DOI: 10.3329/bjsir.v42i3.669
- IZUMI, GAKU ET AL.: '«Fatty acid derivatives and their utilization. IX. Synthesis and properties of high-molecular-weight aliphatic poly(amide-amines)»' KOGYO KAGAKU ZASSHI vol. 72, no. 4, 1969, pages 1018 - 1022, XP008174379 & DATABASE CAS 1969 STN Database accession no. 26185-22-8

## Description

### FIELD OF THE INVENTION

The present invention relates generally to dispersants. More specifically, this invention relates to bio-based dispersants. More particularly, the invention relates to derivatives of natural oils that have a high level of renewable raw materials and are useful as bio-based dispersants.

This invention also relates to methods of making natural oil based dispersants, and specifically to methods of synthesizing natural oil based dispersants, particularly wherein they are made from at least one biologically-derived precursor.

This invention further relates generally to dispersants derived from biomass, and specifically to methods and systems for efficiently making bio-based dispersants from natural and or vegetable or crop oils.

This invention also relates to new and useful non-aqueous liquid pigment dispersions incorporating bio-based dispersants which are easy to handle and produce thorough and effective colorations within target media, particularly as compared to standard solid pigments or high-viscosity liquid pigment dispersions.

The present invention further relates to a class of bio-based dispersants for non-aqueous systems which can disperse pigments in non-aqueous liquids within a short period of time and thereby give dispersions showing good dispersion stability for a prolonged period of time.

Furthermore, the invention relates to dispersants which, when pigments insoluble in organic liquids are dispersed in said organic liquids in the presence thereof, can provide dispersions particularly excellent in flowability and dispersion stability.

The present disclosure is also directed to methods for the preparation of such bio-based dispersants and uses of the bio-based dispersants.

### BACKGROUND OF THE INVENTION

The introduction of solids into liquid media requires high mechanical forces. This depends substantially on the wettability of the solid by the surrounding medium and on the affinity to this medium. For the purposes of reducing these dispersing forces it is conventional to use dispersants facilitating the dispersion. These are mostly surfactants or tensides having an anionic, cationic or a non-ionic structure. These agents are directly applied to the solid or added to the dispersing medium in relatively small amounts.

It is further known that these solids tend to flocculate following the dispersion, which nullifies the work earlier done and leads to serious problems. These problems have been accounted for by the London/van der Waal's forces by which the solids attract each other. For the purposes of counteracting these attractive forces absorption layers must be applied to the solid. This is done by using such tensides.

During and following the dispersion there is an interaction between the surrounding medium and the solid particle, resulting in a desorption of the tenside by exchange for the surrounding medium present in a higher concentration. This medium, however, is not capable in most cases of building up such stable absorption layers, resulting in a crash of the whole system. This becomes apparent by the increase in viscosity in liquid systems, losses of gloss and color shifts in lacquers and coatings as well as insufficient color force development in pigmented synthetics.

To solve this problem, e.g., EP-A 154,678, EP-A 74080, U.S. Pat. No. 4,032,698 and DE-A 24 38 414 propose the use dispersants. These dispersants, however, only lead to a partial solution, particularly with respect to the miscibility without flocculation of different pigments with each other, such as organic pigments and inorganic pigments. Moreover, the pigment pastes prepared by the methods defined tend to interact with the surrounding medium, e.g., after use in lacquers. Consequently, it can be assumed that the absorption layers built up only have insufficient stability against desorption. A number of dispersants proposed in these publications further have the drawback that the storage stability is too poor, which leads to precipitation, phase separation, crystallization, etc. This results in that such products are inhomogeneous and useless in practice after a relatively short time.

It is also known that pigments are widely used as colorants, for example, in paints, varnishes, and inks. Such pigments generally have average particle sizes (diameters) in the range of 0.1 to 10 micrometers, more typically, 1 micrometer or greater. To achieve these particle sizes, mechanical devices are most often used to comminute solid particulate into smaller primary particles. The most common mechanical devices include ball mills, attritions, sand/bead mills, and roll mills. All of these devices require moving parts in order to generate the mechanical forces required to break up the pigment particles. Milling times of several hours are typical, with certain pigments requiring a day or longer in order to break up, or comminute, the particles. Moreover, comminution of the pigment by contact with the milling media results in pigment surfaces exhibiting a high number of surface asperities. Furthermore, contamination of the dispersions from the mechanical parts of the milling equipment can result due to the intimate contact of the pigment with the milling media. Silicon dioxide, a grinding medium, is a common contaminant found after sand milling, for example.

Another disadvantage of mechanical processing of pigments is the large breadth of distribution of particle sizes resulting from such processes. This results in the presence of particles having diameters of one micrometer or greater, even in dispersions where the average particle size is significantly less. For dispersions requiring transparency in the final article, these larger particles lead to unwanted light scattering and are detrimental. The presence of these micrometer sized particles also leads to inherent instability, or tendency to flocculate, in the dispersions.

More stable pigment dispersions can be obtained by chemically altering the pigment. This often results in smaller average particle diameters but has the disadvantages of requiring a chemical pretreatment of the pigment, still requiring mechanical milling, and still providing a dispersion having a wide particle size distribution.

Current pigment dispersants are effective to some degree in dispersing a pigment in a higher concentration in a non-aqueous dispersion medium and in stabilizing the dispersion, but do not offer a satisfactory effect on stabilization of a fine dispersion of the pigment.

The products commonly employed in the prior art i.e., carbon black dispersants in coatings are salts of an acrylic acid copolymer, acetylene diol surfactants, or poly alcohol ethers which fit into various classes of wetting and dispersing agents, (Calbo, Handbook of Coatings Additives, Dekker pg. 516). Such additives could be called on to function as more than a dispersant and can also act in one or more of the following ways: a) to prevent flocculation, b) to prevent hard settling, c) to improve jetness/color/gloss, d) to control viscosity, and/or e) to improve wetting of the base resin.

Various considerations are important in determining the usefulness of any additive as a dispersing agent for use with a carbon black or with other pigments, depending upon the product into which such a dispersion is to be incorporated. When used throughout this application the terms pigment(s) or pigment dispersion(s) are intended to encompass various materials which may be intended to impart either color and/or serve some other function, such as for example the use of carbon black in rubber where, in addition to adding color, such also acts as a reinforcing agent.

One of the most important considerations in determining whether a particular dispersant will be useful for use with a given pigment or pigments when such a pigment is to be used in a paint or coating composition is whether such a dispersant/pigment combination will or will not impart a conductive nature or characteristic to the dried paint film or coating into which it has been added.

The automotive industry is replacing and will continue to replace exterior metal body panels on vehicles with plastic and composite body panels. Some reasons for this change are weight reduction, flexibility of design, and lower tooling costs. The replacement of metal body panels by plastics and composites is not without difficulties.

One problem of note is the electrostatic spray painting of plastics. Electrostatic spray painting is the preferred manner of applying automotive topcoats. Spray painting normally gives the best appearance to the vehicle and the electrostatic technique assures the most economical use of the material. The problem arises because plastics do not paint well electrostatically unless a conductive primer is used.

Amongst the most important considerations for determining the utility of any dispersant to be used in conjunction with conductive carbon blacks are the following: the inherent rheological stability of the dispersion, both alone and when added to a formulated paint; resistance to flocculation of the carbon black/dispersant mixture and in the final paint or coating; and ability to achieve low viscosity at high pigment loadings.

The various prior art references of which the applicants are aware which relate to dispersing agents for pigment additives, such as carbon blacks, suffer from a number of shortcomings. The most significant shortcomings of the carbon black dispersants of the prior art, including those used for conductive carbon blacks, are: high levels of dispersant may be required which tends to detrimentally affect the physical properties of formulated paints, such as adversely affecting the resultant humidity resistance, yellowing upon exposure to UV light, loss of cure in melamine cross-linked systems, and other undesirable effects; inability to prevent reflocculation of carbon black, resulting in the loss of electrical conductivity in dried paint films; and incompatibility of the dispersant with the particular resin system selected for use in the final paint formulation.

Additionally, more and more paints are produced which are water-based and completely free from organic solvents, such as glycol ethers. When toning these paints to the desired colour, use is made to a great extent of pigment dispersions, which can be used both for water-based paint and for paint based on organic solvents. The pigment dispersions are normally composed of pigments, fillers, dispersing agents and an aqueous phase in the form of ethylene glycol, di- and triethylene glycol, propylene glycol, dipropylene glycol, tripropylene glycol and glycerol. In most cases, the dispersing agent is a nonionic surface-active compound or a combination of nonionic and anionic surfactants. For environmental reasons, it is however desirable that the pigment dispersions are solvent-free.

Additionally, it is known that dispersants are required components of many industrial formulations and applications such as paints and coatings, inks, oilfield chemicals, and lubricants. Several distinct classes of dispersants have emerged as being particularly effective in the dispersion of pigments and fillers in both non-aqueous and aqueous formulations. Particularly exemplary dispersants are those derived from certain fatty acids or esters and polyethyleneimine such as those described in U.S. Patent No. 8,168,713. However, the high cost of the polyethyleneimine is a major impediment to their widespread use in many applications. A further disadvantage is that the use of these dispersants is often limited by their solubility in a wide range of polar and non-polar media.

Dispersants and latexes have utility in applications such as paper coatings, colors, paints, and adhesives, as well as coatings for paper, metal, and the pharmaceutical industry. Although dispersants account for only a few percent of the total composition of paints and coating formulations, the dispersants play a critical role in the performance of such paints and coating formulations. The dispersants provide color stability and maximize pigment opacity by increasing the exposed surface area of the pigment particles, thus increasing coverage while reducing costs.

Dispersion is a complex process which involves variables including the chemistries of the solvent, resin, and pigments involved. Changes in these chemistries are associated with changes in the rheology and the resultant dispersant technology. Steric and electrostatic forces can stabilize pigment dispersions and are often accomplished with anionic and nonionic surfactants and their resulting effects on the pigment surface. These surfactants are easy to use, inexpensive, and effective at low concentrations. But, anionic surfactants are pH and salt sensitive. Adsorption of non-ionic surfactants is pH and salt insensitive, but such non-ionic surfactants need to be used in large amounts to be effective.

Other dispersant technologies use hyperdispersants which have higher molecular weights than traditional, surfactant-like dispersants. One type of such hyperdispersants are polymeric dispersants which have an anchoring group in their molecule that absorbs at the surface of the pigments and a polymeric chain that provides a steric stabilization barrier around the pigment particle. Although the polymeric dispersants absorb onto the dispersed pigments, such dispersants provide little wetting and emulsifying properties. Such dispersants are attractive in some water based-formulations because less foaming often results as compared to the surfactant-like dispersants.

Phosphate esters are often used in conjunction with dispersant technologies and are considered auxiliary dispersants since such phosphate esters are not used by themselves. The phosphate esters provide assistance with stabilization through steric interactions with the pigment particles.

Apart from the abilities of wetting and dispersing, dispersants also need to stabilize the suspended particles or the suspended particles will re-agglomerate. This stabilization is critical and difficult to accomplish, but when achieved, provides a colorant with a longer shelf life, improved color, gloss, and color compatibility.

One surfactant that exhibits these desirable dispersant properties is anionic phosphate esters which have a phosphate moiety as a head group. The anionic phosphate esters are synthesized with phosphoric acid derivatives and alcohol and have some residual phosphoric acid resulting in a pH as low as two. Anionic phosphate esters are often available in free acid form. The presence of the phosphate group in a formulation for a wetting or dispersing agent enhances the gloss and color acceptance property of a pigment in paint, reduces a viscosity increase due to aging of the paint, improves surface wetting, and provides a stable dispersion.

With the growing need for more biobased additives that can replace petroleum based products based on the desire for "greener" products, a need exists for biobased products that can be used in dispersants, coatings, and latex type products where the biobased products fulfill all the desired characteristics of the petroleum based counterparts.

Thus, there is a need for lower cost versatile bio-based dispersants that can be utilized to disperse pigments or fillers in a variety of applications.

### OBJECTS OF THE INVENTION

Disclosed are bio-based dispersants. Such bio-based dispersants are also disclosed in WO 2013/126916.

Also disclosed are pigment dispersions incorporating bio-based dispersions.

Also disclosed are pigment dispersions containing bio-based dispersants based on unsaturated and polyunsaturated natural oils that have been reacted via Diels-Alder reaction.

Also disclosed are pigment dispersions containing bio-based dispersants based on unsaturated and polyunsaturated natural oils that have been reacted via the ene reaction.

Also dislcosed are carbon black dispersions containing novel bio-based dispersants.

Also disclosed are carbon black dispersions incorporating bio-based dispersants based on unsaturated and polyunsaturated natural oils that have been reacted via Diels-Alder reaction and ene reactions.

Further disclosed are non-aqueous pigment dispersions incorporating bio-based dispersants based on unsaturated and polyunsaturated natural oils that have been reacted via Diels-Alder reaction and ene reactions.

Also disclosed are mixtures of bio-based dispersants based on unsaturated and polyunsaturated natural oils that have been reacted via Diels-Alder reaction and ene reactions.

Still, also disclosed are pigment dispersions having a very high tinctorial strength and brilliance, an excellent levelness and covering power in opaque applications.

A object of the invention is to provide thermoplastic and/or thermosetting polymer compositions, or polymer compositions transformable at low or high temperature, which are the basis for plastics materials with improved physical and chemical properties such as mechanical, thermal, dielectric, and esthetic properties comprising at least one bio-based dispersing agent.

These and other objects of the present invention will more readily become apparent from the description and examples which follow.

### BRIEF DESCRIPTION OF THE FIGURES

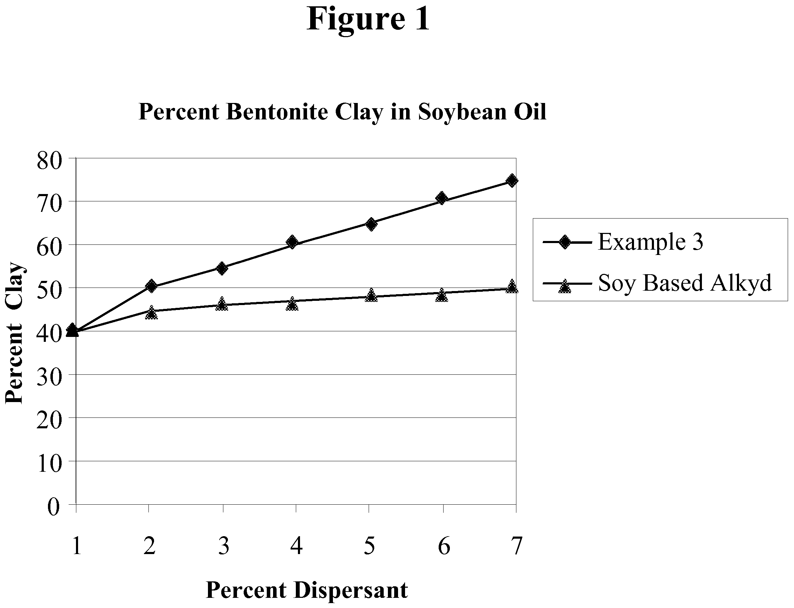

Figure 1 illustrates that clay loadings of up to 75 percent were achieved with a soybean oil gel of Example 3 while loadings of only 50 percent were achieved with the standard soy based alkyd resin.

### SUMMARY OF THE INVENTION

Applicant has discovered that reaction products of modified natural oils and natural oil derivatives with alkanolamines or alkoxylated alkanolamines provide compositions that have many advantages over dispersants provided by prior art. The compositions are prepared by a simple, economical process from low cost precursors, and they surprisingly exhibit superior dispersing characteristics compared to higher cost dispersants.

Disclosed is a bio-based dispersant comprising the reaction product of a natural triglyceridic oil, fatty acid oil or fatty acid oil derivative wherein said natural triglyceridic oil, fatty acid oil, or fatty acid oil derivative contains greater than about 80 percent of unsaturated or conjugated unsaturated fatty acids with a substrate capable of undergoing an ene or Diels Alder reaction to form an adduct that is further esterified and/or neutralized with an alkanolamine or alkoxylated alkanolamine.

Also disclosed is a pigment dispersion comprising: (a) a pigment; (b) a solvent; and (c) a bio-based dispersing agent comprising the reaction product of a natural triglyceridic oil, fatty acid oil or fatty acid oil derivative wherein said natural triglyceridic oil, fatty acid oil, or fatty acid oil derivative containing greater than about 80 percent of unsaturated or conjugated unsaturated fatty acids with a substrate capable of undergoing an ene or Diels Alder reaction to form an adduct that is further esterified and/or neutralized with an alkanolamine or alkoxylated alkanolamine.

The instant invention provides a polymer dispersion comprising: (a) a thermoplastic resin selected from the group consisting of polyetherketone, polyethylene or a polypropylene; (b) a solvent; and (c) a bio-based dispersing agent comprising the reaction product of a natural triglyceridic oil, fatty acid oil or fatty acid oil derivative wherein said natural triglyceridic oil, fatty acid oil, or fatty acid oil derivative containing greater than 80 percent of unsaturated or conjugated unsaturated fatty acids with a substrate capable of undergoing an ene or Diels Alder reaction to form an adduct that is further esterified and/or neutralized with an alkanolamine or alkoxylated alkanolamine.

Also disclosed are bio-based dispersants selected from the group consisting of compounds of the formula: 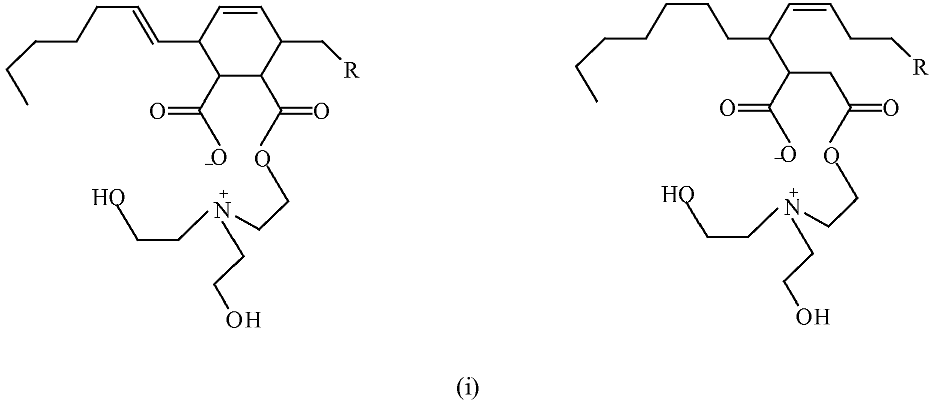 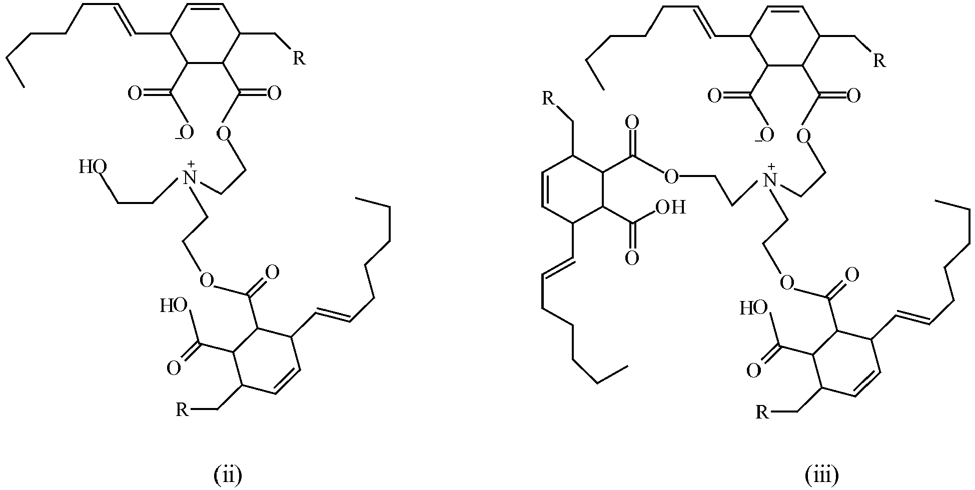 where R represents the additional carbon and functional moieties associated with a natural oil.

### DETAILED DESCRIPTION OF THE PRESENT INVENTION

Disclosed is an unsaturated natural oil or derivative thereof modified by a process in which the double bonds and/or conjugated double bonds of the triglyceride oil chains are reacted via thermal condensation with an unsaturated substrate which is capable of undergoing an "ene" reaction or an Diels Alder reaction with the sites of unsaturation resulting in the formation of an adduct of the triglyceride oil. The adduct formed is preferentially an anhydride and the substrate reacted in this manner with the triglyceride oil is preferably maleic anhydride. The term "maleation" has been historically applied to the reaction of natural unsaturated oils, fatty acids and their derivatives with maleic anhydride. Functionaliztion of triglyceride oils by this method is well known in the art and is described in US 2,033,131, 2,033,132 and 2,063,540. These adducts are generally referred to in the literature as "maleated oils" or maleinized oils". While maleic anhydride is the preferred unsaturated substrate for adduct formation due to it's low cost and stability, it is well known to those skilled in the art that any α, β-unsaturated molecule capable of undergoing a thermal "ene" reaction or a Diels Alder reaction may be employed. Specific non-limiting examples include itaconic acid, itaconic anhydride, acrylic and methacrylic acid, fumaric acid, aconitic acid, citraconic acic, maleimide, maleamic acid and the like.

The bio-based dispersants are made as follows: In a first step, 2000 parts by weight of the natural oil and either one or two molar equivalents (based on the natural oil) of maleic anhydride are charged to a 3 L four-neck round-bottom flask. These amounts correspond to 11.0 and 22.0 weight percent of maleic anhydride based on the weight of the natural oil respectively. Higher or lower molar ratios of maleic anhydride may be used in order to adjust water or oil solubility of the dispersant. The contents of the flask are gradually heated to 210° C with agitation under a nitrogen sparge. The reaction mixture is held at this temperature until no free maleic anhydride is detected in the reaction mixture by GC analysis. The reaction mixture was cooled to 60° C and triethanolamine or an alkoxylated triethanolamine at the desired molar ratio to anhydride functionality is slowly added. The reaction mixture is held at 80° C for 1 hour or until no unreacted maleic anhydride was observed in the Infrared Spectrum of the reaction mixture and the dispersant obtained was discharged.

The natural oils or natural oil derivatives that may be used in the herein disclosed method include any triglyceride oil or derivative of such that contains significant portions of unsaturated fatty acids. Natural triglyceride oils containing both non-conjugated and conjugated double bonds are suitable. Non-limiting examples of suitable triglyceride oils include soybean oil, linseed oil, safflower oil, sunflower oil, avocado oil, rapeseed oil, castor oil, tall oil, rosin oil and tung oil. The fatty acids employed in the present invention generally correspond to the fatty acid components of the triglyceride oil and include oleic acid, linoleic acid, linolenic acid, tall oil fatty acid, gadoleic acid, ricinoleic acid and the like.

Derivatives of these fatty acids such as esters, amides etc. may also be used. These include but are not limited to methyl esters, ethyl esters, esters derived from longer chain alcohols, generally C₄ - C₂₂ alcohols, esters derived from polyhydroxy alcohols such as glycerol, diglycerol and polyglycerols, pentaerythritol, dipentaerthyritol, trimethylolpropane, ditrimethylolpropane and the like. Also useful are diesters of glycols such as ethylene glycol, propylene glycol, butylene glycol and the like as well as polyols such as polyethylene glycol, polypropylene glycol, polybutylene glycol and the like.

The maleated natural oil or natural oil derivative is further reacted with one or more molar equivalents of alkanolamine or alkoxylated alkanolamine in an esterification and/or neutralization reaction to provide compositions of formula (i), (ii), and (iii) wherein the alkanolamine is triethanolamine, and compositions of formulas (i), (ii), (iii) where the alkanolamine is an alkoxylated triethanolamine of formula (iv). 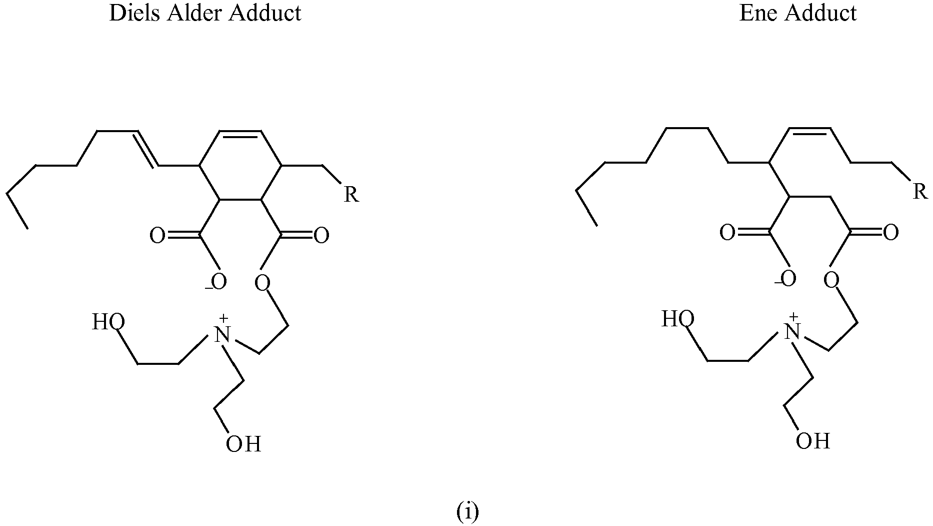 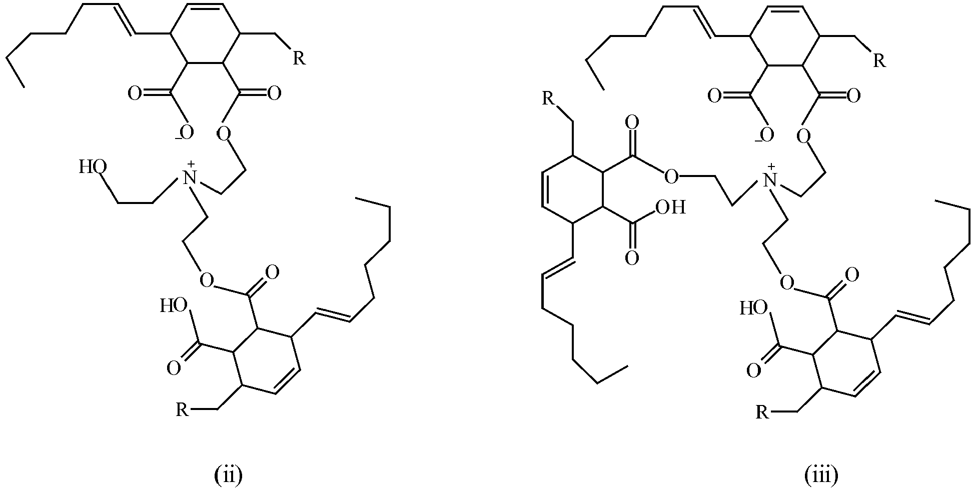 where R represents the additional carbon and functional moieties associated with a natural oil. The R moiety is basically the remaining part of the triglyceride oil, fatty acid or fatty acid derivative that has not reacted with the unsaturated substrate that is used for the ene or Diels Alder reaction.

To understand what is meant by the additional carbon and functional moieties the structure of soybean oil is depicted below:

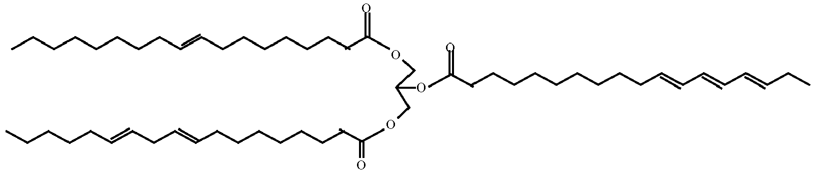

In the case of the above and all conjugated oils, the R represents all carbon and functional moieties to the left of the conjugated sites.

In the cases of neutralization with an alkoxylated triethanolamine, the alkoxylated triethanolamine has the following structural formula 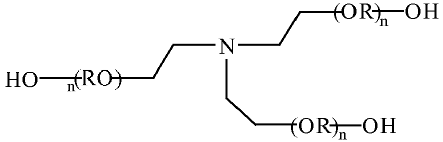 where R= -CH₂-CH₂- or 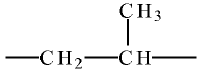 , and n=0-200.

The amount of triethanolamine or alkoxylated triethanolamine reacted with the maleated oil or derivative depends on the desired final properties of the dispersant. It will be recognized by those skilled in the art that properties such as pH and solubility may be controlled by the type and amount of alkanolamine reacted with the functionalized oil or derivative. It will also be recognized by those skilled in the art that such compositions will actually be mixtures; the relative amounts of the component formulas depending upon the molar ratios of the functionalized oil to alkanolamine.

The use of alkoxylated alkanolamines allows control of the water and oil solubility of the dispersants; the more water soluble ethylene oxide derivatives being more hydrophilic, and the more oil soluble alkylene oxide derivatives such as the propylene oxide and butylene oxide derivatives being more lipophilic. The ratio of the alkanolamine or alkoxylated alkanolamine to the functionalized oil allows control of the pH of the dispersant and thus the composition can be tailored to specific pigments or fillers.

In the present specification, the term "non-aqueous" denotes a composition into which no water has been specifically introduced. Due to the possibility of atmospheric water being introduced through exposure to a relatively humid environment, this term does not rule out the potential for any water to be present through such a manner. The term "liquid dispersion" is intended to encompass any composition which is present in a fluid state (i.e., possessing a viscosity of below about 10,000 centipoise at standard temperature and pressure).

The non-aqueous solvent usable in the present invention varies depending upon the use of the pigment dispersion of the present invention. For instance, examples of the non-aqueous solvent for use in paints are usual organic solvents such as esters, ethers, ketones, alcohols, and aromatic solvents. Examples of other non-aqueous solvent include alcohols such as ethanol, isopropanol, butanol, as well as C₅-C₂₂ alcohols, the glycol ethers such as ethylene glycol monoethyl ether, diethylene glycol monoethyl ether, diethylene glycol dimethyl ether and dipropylene glycol monoethyl ether, glycol mono ether acetates such as ethylene glycol monoethyl ether acetate, propylene glycol monomethyl ether acetate and diethylene glycol monoethyl ether acetate, ketones such as cyclohexanone and methyl ethyl ketone, amides such as N,N-dimethylacetamide and N-methylpyrrolidone, lactones such as .gamma.-butyrolactone, and acetic acid esters such as butyl acetate and isopropyl aceate. Preferable as the non-aqueous solvent for use in printing inks, particularly offset printing inks are lipophilic solvents such as vegetable oils and mineral oils.

In applications where resins are present, plasticizers are an important component of the formulation. Examples of plasticizers that are suitable include benzyl butyl phthalate, dibutyl phthalate, triphenyl phosphate, 2-ethyl hexyl benzyl phthalate and dicyclohexyl phthalate. Other well-known plasticizers which may be employed include dinonylphthalate, diisononylphthalate, diallyl phthalate, dibenzyl phthalate, butyl cyclohexyl phthalate, mixed benzoic acid and fatty oil acid esters of pentaerythritol, poly(propylene adipate)dibenzoate, diethylene glycol dibenzoate, tetrabutylthiodisuccinate, butyl phthalyl butyl glycolate, acetyl tributyl citrate, dibenzyl sebacate, tricresyl phosphate, toluene ethyl sulfonamide, the di 2-ethylhexyl ester of hexamethylene diphthalate, and di(methylcyclohexyl)phthalate. The particular plasticizer and the amount thereof used are chosen in accordance with the demand for compatibility.

The pigment or the disclosed may be selected from inorganic pigments (such as carbon black pigments, e.g., furnace blacks, electrically conductive carbon black pigments, extender pigments and corrosion inhibitive pigments); organic pigments; dispersed dyes; and mixtures thereof. Examples of organic pigments that may be present in the pigment dispersion include, but are not limited to, perylenes, phthalo green, phthalo blue, nitroso pigments, manoazo pigments, diazo pigments, diazo condensation pigments, basic dye pigments, alkali blue pigments, blue lake pigments, phloxin pigments, quinacridone pigments, lake pigments of acid yellow 1 and 3, carbazole dioxazine violet pigments, alizarine lake pigments, vat pigments, phthaloxy amine pigments, carmine lake pigments, tetrachloroisoindolinone pigments and mixtures thereof. Inorganic pigments that may be present in the pigment dispersion, include, for example, titanium dioxide, electrically conductive titanium dioxide, and iron oxides, e.g., red iron oxides, yellow iron oxides, black iron oxides and transparent iron oxides. Extender pigments that may be present in the pigment dispersion include, but are not limited to, silicas, clays, alkaline earth metal sulfates and carbonates, such as calcium sulfate, magnesium sulfate, barium sulfate, and calcium carbonate. The pigment dispersion may contain corrosion inhibitive pigments, such as aluminum phosphate and calcium modified silica. Mixtures of organic and inorganic pigments are also suitable for making the disclosed dispersions.

Pigment blacks with an average primary particle diameter of 8 to 80 nm, preferably 10 to 35 nm, and a DBP number of 40 to 200 ml/100 g, preferably 60 to 150 ml/100 g, can be used as the carbon black. In a preferred embodiment of the invention, gas blacks with an average primary particle diameter of 8 to 30 nm, preferably 10 to 25 nm, can be used.

The pigment dispersions contain 1 to 85% by weight, preferably 30 to 80% by weight, of pigment, 0.5 to 35% by weight, preferably 1 to 20% by weight, of the bio-based dispersant, 0 to 20%, preferably 0 to 5%, of additional nonionic or anionic surface-active agents if necessary, and 25 to 60% by weight of a solvent.

The amount of dispersants depends on the specific materials employed and the concentration of pigment in the dispersion required. For inorganic pigments, such as titanium dioxide and iron oxide pigment, the amount used is typically in the range 0.02 to 20%, commonly 0.05 to 10% and more usually 0.1 to 5.5%, by weight of the pigment; for organic pigments such as phthalocyanines, somewhat higher levels of dispersant may be used, typically in the range 0.02 to 50%, more usually from 0.1 to 30%, by weight of the pigment; and for carbon black the amount of dispersant is typically in the range 0.02 to 30%, more usually from 0.1 to 20%, by weight of the pigment.

When incorporated into end use products such as paints or surface coatings typical pigment levels on the final product will be from about 0.02 to about 40%, particularly about 0.1 to about 25%, pigment by weigh based on the total paint or surface coating. Where coloured inorganic pigments are used, the levels will typically be from about 0.05 to about 25%, particularly about 0.2 to about 15%, for white pigments, particularly titanium dioxide, the pigment may be present to provide opacity and not just colour and will often be present at concentrations e.g. in base paint formulations, of up to 25%, typically from 0.2 to 25%, by weight; for organic pigments, especially phthalocyanine pigments, the levels will typically be up to about 10% typically from about 0.05 to about 8%, particularly about 0.1 to about 5%; and for carbon black the levels will typically be from about 0.05 to about 10%, particularly about 0.2 to about 5%.

The non-aqueous pigment dispersion composition is prepared by adding a pigment to a non-aqueous solution of a pigment dispersant, disaggregating and dispersing the pigment in the solution by means of a dispersing machine such as roll mill, ball mill or sand mill, diluting the resultant dispersion to a desired concentration and removing larger particles therefrom by way of centrifugation, Scharples-type centrifugation and filtration. If a desired particle-size distribution cannot be obtained by the first particle classification process, the dispersing process and particle classification process are repeated until the desired particle-size distribution is obtained. In most instances, if the proportion of particles impassable through a sieve having a mesh size of 300 nm is not greater than 30%, there is no need for particle classification. Thus, the pigment contained in the dispersion has a median particle size of not greater than 250 nm, preferably not greater than 200 nm with not greater than 30% of the pigment particles being impassable through the sieve having a mesh size of 300 nm.

Also, the pigment dispersion can be produced by first preparing a pigment-free mixture of the surface-active compounds, the non-aqueous solvent, the antifoaming agents and any other additives, and subsequently adding the pigment portion which is dispersed in the mixture. The dispersion can be carried out by means of a dissolver or grinder, for instance a ball grinder or roller mill.

The dispersion is preferably storage stable. By this term, it is intended that the inventive dispersion will remain in a fluid state with substantially no precipitation or reagglomeration of pigment for at least 60 days while being continuously exposed to a temperature of at least 50° C. Such a test is one manner of reproducing long-term storage conditions and thus is not intended as being the sole limitation of temperature within this invention. One of ordinary skill in this art would appreciate the need to provide a modified test of this nature. Thus, the dispersions must merely exhibit substantially no precipitation and retention of its fluid state (low viscosity) after exposure to high temperature storage for 60 days.

The pigment dispersions can be employed for all purposes and are excellently suitable for the production of emulsion paints based on polyvinyl acetate, polyvinyl acetate copolymers, styrene-butadiene copolymers, polyvinyl propionates, acrylic and methacrylic acid ester polymers, saponified alkyd resins and oil emulsions; for the production of wallpaper paints based on cellulose derivatives such as methylcellulose, hydroxymethylcellulose and carboxymethylcellulose, and for the production of printing inks which contain, as binders, mainly saponified natural resins, such as shellac, saponified water-soluble synthetic resins or acrylate binder solutions.

The bio-based dispersant find uses in many applications. In many applications it dispersed pigment, polymer, plasticizer, and plastisols. In both applications, lower viscosity and higher color yield for the pigment resulted. When lower viscosity is achieved, it allows either increased shear of the particulate matter added, be it pigment or dyes. In the case of pigment, this offers increase color strength; thus, saving money for the end user. The dispersant does not coalesce the polymer, but lowers the particle size of it, which makes for a more efficient coating. This offers what is known as 'plate out' prevention with injection plastisol machines.

The bio-based dispersants also work with Pigment Red 57:1, diiso-nonyl phthalate, and florescent pigments, which are made with formaldehyde resins, benzoquinoneimines, and melamine formaldehyde. They are essentially dyed polymers. The also work with any oil, or liquid plastic (plastizol) dispersion, where water is not present, and pigment, dye, or any particulate matter has to be dispersed. This includes inks, paints, any coating. Possibilities are solvent-borne resins, which include Alkyds, Alkyd Copolymers, Oil Modified Urethanes (OMU), Polyesters and Solution Acrylics.

Although Applicant does not wish to be bound by theoretical explanations of interfacial phenomena, it is believed that the dispersant works by drastically lowering the interfacial tension of the polymer/plasticizer and the pigment. When shear is applied, the polymer/plasticizer and pigment breaks into smaller particles. Since the dispersant has a high affinity for low HLB type polymers, steric hindrance takes place to keep the particles evenly spaced in a lower energy state. This allows for further development of the pigment color using the conventional dispersing equipment. After a lower viscosity is attained, many options exist for the user. Higher solids can be gained on pigment, resin, or plasticizer, not to mention the possible increase in color yield.

In another preferred embodiment of the invention, additives useful for making synthetic resin products are dispersed into the resins using the dispersant of the present invention. The dispersant of the invention may be added or injected directly into a polymer melt or into a polymer solution using a solvent. The dispersant is added in the range of 0.01% to 30% by weight of the resin and the additive is present in the range of 0.01% to 40% by weight.

The additives that can be added to the resins are selected from the group consisting of (i) one or more mineral fillers, organic fillers of natural or synthetic origin or a mixture thereof wherein said one or more mineral fillers is selected from the group consisting of titanium dioxide, natural calcium carbonate, precipitated calcium carbonate, magnesium carbonate, zinc carbonate, dolomite, lime, magnesia, barium sulfate, calcium sulfate, aluminum hydroxide, magnesium hydroxide, silica, wollastonite, clays, talc, mica, solid glass spheres, hollow glass spheres, and metal oxides and wherein said organic fillers are selected from the group consisting of organic materials of natural and synthetic origin, and (ii) one or more additives selected from the group consisting of antioxidants, metal deactivators, light stabilizers, pvc stabilizers, plasticizers, lubricants, processing aids, impact modifiers, fiber reinforcements, flame retardants, antistatic agents, fluorescent whitening agents, biostabilizers, antimicrobials, chemical blowing agents, organic peroxides, nucleating agents, polymerization catalysts, grafting catalysts, thermal stabilizers, photochemical stabilizers, shrink-preventive agents, antistatic agents, mold-release agents, glass fibers, and mineral thickeners and mixtures thereof, and mixtures of (i) and (ii).

The thermal stabilizing agent is an antioxidant and is selected from the group consisting of: tetrakis[methylene 3-(3',5'-di-tert-butyl-4'-hydroxyphenyl)-propionate]-methane, octadecyl 3-(3',5'-di-tert-butyl-4-hydroxyphenyl)propionate, 1,3,5-trimethyl-2,4,6-tris-(3,5-di-tert-butyl)-4-(hydroxyl-benzyl)benzene, bis(2,4-di-tert-butyl-phenyl)pentaerythritol diphosphite, tris(mono-nonylphenyl)phosphite, 4,4'-butylidene-bis(5-methyl-2-tert-butyl)phenol, tris(3,5-di-tert-butyl-4-hydroxybenzyl)isocyanurate, tris-nonylphenyl phosphite, distearyl pentaerythritol diphosphite, tetrakis-(2,4-di-tert-butylphenyl)-4,4'-biphenylene diphosphonite, tris-(2,3-di-tert-butylphenyl)-phosphite, butylated hydroxy toluene, dicetyl thiodipropionate, dimyristyl thiodipropionate, and poly(1,4-cyclohexylene-3,3'-thiodipropionate, partially terminated with stearyl alcohol, as well as mixtures of any two or more thereof.

Typical antistatic agents are selected from the group consisting of glycerol monostearates, ethoxylated amines, polyethylene glycols, and quaternary ammonium compounds, as well as mixtures of any two or more thereof.

The coupling agents are selected from the group consisting of silanes titanates, chromium complexes, carboxyl-substituted polyolefins, carboxyl-substituted acrylates, and paraffins, as well as mixtures of any two or more thereof.

The UV stabilizers are selected from the group consisting of: 2-hydroxy-4-octoxybenzophenone, 2-hydroxy-4-isooctoxy-benzophenone, 4-hydroxy-4-n-dodecycloxybenzo-phenone, 2-(3-di-tert-butyl-2-hydroxy-5-methylphenyl-5-chlorobenzyltriazole, 2-(2-hydroxy-3,5-di-tert-amylphenyl)-benzotri-azole, para-tert-butylphenyl salicylate, 2,4-di-tert-butylphenyl-3,5-di-tert-butyl-4-hydro-xybenzoate, nickel bis-ortho-ethyl(3,5-di-tert-butyl-4-hydroxybenzyl) phosphonate, and 2,2',6,6'-tetramethyl-4-piperidinyl sebacate, as well as mixtures of any two or more thereof.

The flame retardant is selected from the group consisting of: decabromodiphenyl oxide, dodecachlorodimethane dibenzocyclooctane, ethylene bis-dibromo norbornane dicarboxamide, ethylene bis-tetra-bromophthalimide, and antimony trioxide, as well as mixtures of any two or more thereof.

The metal deactivating agent is selected from the group consisting of: oxalyl bis-(benzylidene hydrazide), and 2,2'-oxamido bis-(ethyl 3-(3,5-di-tert-butyl-4-hydroxyphenyl) propionate, as well as mixtures of any two or more thereof.

The nucleating agent is selected from the group consisting of sodium benzoate, diphenyl phosphinic acid, the magnesium, sodium, calcium, and aluminum salts of diphenyl phosphinic acid, phenyl phosphinic acid, the magnesium, sodium, calcium, and aluminum salts of phenyl phosphinic acid, phenyl phosphorous acid, and the magnesium, sodium, calcium, and aluminum salts of phenyl phosphorous acid, as well as mixtures of any two or more thereof.

The resins are selected from the group consisting of: (a) a thermoplastic resin selected from the group consisting of: (i) low- or high density polyethylene, linear or branched, (ii) homo- or copolymeric polypropylenes, and polyetherketones.

The method of manufacturing filled polymer compounds which are preferably flowable and homogeneous (i.e., well mixed) according to the invention, is characterized in that the inventive dispersing agent is added to the mineral and/or organic fillers prior to their introduction to the resin, or to the resin prior to or after the introduction of said fillers to the resin.

The polymer compositions of the invention may be employed in any method of forming or processing of thermoplastics, such as extrusion, injection molding, calendering, etc.

### EXAMPLES

The following examples are intended to demonstrate the usefulness of preferred embodiments of the present invention.

Examples 1-10 are not according to the invention.

### EXAMPLES 1 -7

The following general method was used to prepare the dispersants listed in Table 1. In a first step, 2000g of the natural oil and either one or two molar equivalents (based on the soybean oil) of maleic anhydride were charged to a 3 L four-neck round-bottom flask. These amounts correspond to 11.0 and 22.0 weight percent of maleic anhydride based on the weight of the soybean oil respectively. Higher or lower molar ratios of maleic anhydride may be used in order to adjust water or oil solubility of the dispersant. The contents of the flask were gradually heated to 210° C with agitation under a nitrogen sparge. The reaction mixture was held at this temperature until no free maleic anhydride was detected in the reaction mixture by GC analysis. The reaction mixture was cooled to 60° C and triethanolamine or an alkoxylated triethanolamine at the desired molar ratio to anhydride functionality was slowly added. The reaction mixture was held at 80° C for 1 hour or until no unreacted maleic anhydride was observed in the Infrared Spectrum of the reaction mixture and the dispersant obtained was discharged.

**Table 1.**

| **Example** | **Percent Maleic Anhydride** | **Alkanolamine** | **Molar Ratio Amine/Maleated Oil** | **pH** | **Solubility** |
|---|---|---|---|---|---|
| 1) Soybean Oil | 11 | TEA | 0.67/1.0 | 6.9 | Water Dispersible |
| 2) Soybean Oil | 11 | TEA | 1.0/1.0 | 7.8 | Water Soluble |
| 3) Soybean Oil | 11 | TEA | 0.33/1.0 | 4.8 | Oil Soluble |
| 4) Soybean Oil | 11 | POP (40)TEA | 0.5/1.0 | 6.5 | Oil Soluble |
| 5) Soybean Oil | 11 | POE (40)TEA | 1.0/1.0 | 6.5 | Water Soluble |
| 6) Soybean Oil | 22 | TEA | 1.0/1.0 | 7.2 | Water Soluble |
| 7) Soybean Oil | 22 | POP (40)TEA | 1.0/1.0 | 7 | Oil Soluble |

### Example 8

### Clay Dispersions

A dispersant as prepared in Example 1 was utilized as a dispersant for the preparation of stable dispersions of bentonite clay in soybean oil which are utilized as fillers in heat set inks. A soy based alkyd resin, commonly used in the industry was used as a control or comparative example. As shown in Figure 1, clay loadings of up to 75 percent were achieved with a soybean oil gel of Example 23 while loadings of only 50 percent were achieved with the standard soy based alkyd resin.

### EXAMPLE 9

### Titanium Dioxide Dispersions

A dispersant as prepared in Example 4 was utilized in the preparation of high solids Titanium Dioxide dispersions in 80/20 Isopropanol/Isopropyl acetate solvent mixture. A standard polyethyleneimine dispersant and a standard polycarboxylic acid dispersant known to be exemplary dispersants in the art were used as comparative examples. As shown in Table 2, stable pourable dispersions containing Titanium Dioxide loadings of only 50-60 percent were achieved with the comparative dispersants, whereas stable, pourable dispersions containing loadings of up to 80 percent were achieved with the dispersant of Example 4.

**Table 2.**

| **Dispersants** | **% TiO₂** | **DispersionStability** | **Dispersion Viscosity(cP)** |
|---|---|---|---|
| Example 4 | 70 | Excellent | 130 |
| Example 4 | 80 | Excellent | 150 |
| Polyethyleneimine based dispersant | 50 | Good | 7000 |
| Polyethyleneimine based dispersant | 60 | Poor | Viscous Paste |
| Polyethyleneimine based dispersant | 70 | NA | Thick Paste |
| Polycarboxylic acid dispersant | 50 | Good | 9000 |
| Polycarboxylic acid dispersant | 60 | NA | Thick Paste |

Dispersions were aged at ambient temperature for 1 month; excellent stability meaning no pigment separation or settling. Some pigment settling occurred after 2 weeks with the other dispersants.

### EXAMPLE 10

### Pigment Dispersions

A dispersant as prepared in Example 1 was utilized in the preparation of dispersions of various pigments in an unsaturated polyester resin. A carboxylic acid alkylammonium salt based dispersant, known as an exemplary dispersant in the art, was used for comparative purposes. As shown in Table 3, the dispersant of Example 1 gave a 50 % reduction in the dispersion viscosity, at the same dispersant level, as compared to the control dispersant at 30 percent pigment loadings for several pigments. The substantial reduction in viscosity of the dispersions at the same pigment loading represents a substantial improvement over the prior art and further demonstrates the usefulness of the natural oil based gels of the present invention.

**Table 3.**

| **Dispersant** | **Pigment** | **Percent Viscosity Reduction** |
|---|---|---|
| | | |
| Example 1 | Yellow 83 | 50 |
| Example 1 | Red 48:2 | 50 |
| Example 1 | Black 330 | 50 |
| Alkylammonium Carboxylate Dispersant | Red 48:2 | 0 |
| Alkylammonium Carboxylate Dispresant | Yellow 83 | 0 |
| Alkylammonium Carboxylate Dispersant | Black 83 | 0 |

### EXAMPLE 11

### Polymer Dispersions

Polymer dispersions in aqueous and non-aqueous solvents were prepared with the dispersant of Example 1 and polyethylene, polypropylene and polyetheretherketone to produce dispersions having excellent stability.

## Claims

1. A polymer composition comprising: (a) a thermoplastic resin selected from the group consisting of a polyetherketone, a polyethylene or a polypropylene; and (b) a bio-based dispersing agent comprising the reaction product of a natural triglyceridic oil, fatty acid oil or fatty acid oil derivative wherein said natural triglyceridic oil, fatty acid oil, or fatty acid oil derivative containing greater than 80 weight percent of unsaturated or conjugated unsaturated fatty acids with a substrate being an α, β unsaturated molecule capable of undergoing an ene or Diels Alder reaction to form an adduct that is further esterified and/or neutralized with an alkanolamine or alkoxylated alkanolamine.

2. The polymer composition of claim 1 , wherein said natural oil is selected from the group consisting of soybean oil, linseed oil, safflower oil, sunflower oil, avocado oil, rapeseed oil, castor oil, tall oil, rosin oil and tung oil.

3. The polymer composition of claim 1, where the substrate is an unsaturated anhydride capable of undergoing the Diels-Alder reaction or the ene reaction.

4. The polymer composition of claim 3, where the substrate is maleic anhydride.

5. The polymer composition of claim 1, where the alkanolamine is triethanolamine or an alkoxylated triethanolamine.

6. The polymer composition of claim 3, where the substrate is methyl maleic anhydride.

## Patentansprüche

1. Polymerzusammensetzung, umfassend: (a) ein thermoplastisches Harz, das aus der Gruppe bestehend aus einem Polyetherketon, einem Polyethylen oder einem Polypropylen ausgewählt ist; und (b) ein Dispergiermittel auf Biobasis, umfassend das Reaktionsprodukt eines natürlichen Triglyceridöls, Fettsäureöls oder Fettsäureölderivats, wobei das natürliche Triglyceridöl, Fettsäureöl oder Fettsäureölderivat mehr als 80 Gew.-% ungesättigte oder konjugierte ungesättigte Fettsäuren enthält, mit einem Substrat, das ein α,β-ungesättigtes Molekül ist, das in der Lage ist, einer En- oder Diels-Alder-Reaktion unterzogen zu werden, um ein Addukt zu bilden, das weiter verestert und/oder mit einem Alkanolamin oder alkoxylierten Alkanolamin neutralisiert wird.

2. Polymerzusammensetzung nach Anspruch 1, wobei das natürliche Öl aus der Gruppe bestehend aus Sojaöl, Leinöl, Safloröl, Sonnenblumenöl, Avocadoöl, Rapsöl, Rizinusöl, Tallöl, Kolophoniumöl und Tungöl ausgewählt ist.

3. Polymerzusammensetzung nach Anspruch 1, wobei das Substrat ein ungesättigtes Anhydrid ist, das in der Lage ist, der Diels-Alder-Reaktion oder der En-Reaktion unterzogen zu werden.

4. Polymerzusammensetzung nach Anspruch 3, wobei das Substrat Maleinsäureanhydrid ist.

5. Polymerzusammensetzung nach Anspruch 1, wobei das Alkanolamin Triethanolamin oder ein alkoxyliertes Triethanolamin ist.

6. Polymerzusammensetzung nach Anspruch 3, wobei das Substrat Methylmaleinsäureanhydrid ist.

## Revendications

1. Composition polymère comprenant: (a) une résine thermoplastique choisie dans le groupe constitué par une polyéthercétone, un polyéthylène ou un polypropylène; et (b) un agent dispersant à base biologique comprenant le produit de réaction d'huile triglycéride naturelle, d'huile d'acide gras ou d'un dérivé d'acide gras, où ladite huile triglycéride naturelle, ladite huile d'acide gras ou ledit dérivé d'acide gras contiennent plus de 80% en poids d'acides gras insaturés ou insaturés conjugués avec un substrat qui est une molécule insaturée α, β à même de subir une réaction d'ène ou de Diels Alder pour former un produit d'addition qui est ensuite estérifié et/ou neutralisé par une alcanolamine ou une alcanolamine alcoxylée.

2. Composition polymère selon la revendication 1, dans laquelle ladite huile naturelle est choisie dans le groupe constitué par de l'huile de soja, de l'huile de lin, de l'huile de carthame, de l'huile de tournesol, de l'huile d'avocat, de l'huile de colza, de l'huile de ricin, du tallöl, de l'huile de résine et de l'huile d'abrasin.

3. Composition polymère selon la revendication 1, dans laquelle le substrat est un anhydride insaturé à même de subir la réaction de Diels-Alder ou la réaction d'ène.

4. Composition polymère selon la revendication 3, dans laquelle le substrat est de l'anhydride maléique.

5. Composition de polymère selon la revendication 1, dans laquelle l'alcanolamine est la triéthanolamine ou une triéthanolamine alcoxylée.

6. Composition de polymère selon la revendication 3, dans laquelle le substrat est de l'anhydride méthylmaléique.
